# EUROPÄISCHE PATENTANMELDUNG

(11) **EP 1 236 516 A2**
(43) Veröffentlichungstag der Anmeldung: **04.09.2002**
(21) Anmeldenummer: 02001645.7
(22) Anmeldetag: 24.01.2002
(51) Int. Cl.: B05B 11/00, A61M 15/08

(54) **Spender für Medien**

(30) Priorität: 28.02.2001 DE 10110742
(71) Anmelder: ING. ERICH PFEIFFER GMBH, 78315 Radolfzell (DE)
(72) Erfinder: Helmlinger, Michael, 78315 Radolfzell (DE)
(74) Vertreter: Patentanwälte , Ruff, Wilhelm, Beier, Dauster & Partner

(57) **Zusammenfassung**

Derartige Spender müssen, damit sie durch einen Benutzer, beispielsweise ein Benutzer der an rheumatuider Arthritis leidet und dessen Fähigkeit zum Manipulieren von Gegenständen unter Umständen eingeschränkt ist, leicht greif- bzw. bedienbar sein. Darüber hinaus sollen die Spender einen einfachen und kostengünstigen Aufbau aus möglichst wenigen Einzelteilen besitzen.

Der Spender ist am Austrag von Medien, insbesondere Fluiden, beispielsweise einer Flüssigkeit geeignet, die vorzugsweise einen pharmazeutischen Wirkstoff - wie sie in beispielhafter Weise vorstehend genannt wurden - enthalten. Bei dem Spender erfolgt der Austrag des Mediums in wenigstens zwei propotionierten Teilchargen dadurch, dass ein Betätigungsmittel betätigt wird. Durch das Betätigen des Betätigungsmittels wird eine Relativbewegung zwischen dem Betätigungsmittel und einem eine Auftragsöffnung des Spenders aufweisenden Gehäuses erzeugt. Gemäss der Erfindung ist eine elastisch verformbare Materialbrücke (21) vorgesehen. Mittels der elastisch verformbaren Materialbrücke (21) ist das Betätigungsmittel am Gehäuse gehalten.

## Beschreibung

Die vorliegende Erfindung betrifft einen Spender für Medien, insbesondere für mindestens einen pharmazeutischen Wirkstoff enthaltende Fluide, vorzugsweise Flüssigkeiten.

Derartige Spender für Medien, bei denen meistens ein zerstäubter Austrag des Mediums, beispielsweise in dosierter (portionierter) Form erfolgt, ist für die Applikation von unterschiedlichsten Medien an unterschiedlichsten Orten bekannt. Insbesondere ist es bekannt, einen zerstäubten Austrag des Mediums im Bereich der Nase vorzunehmen, um Medikamente in die Nasenschleimhäute zu applizieren. Dabei kann es sich um alle Arten von Medikamenten handeln, deren pharmazeutische Wirkstoffe über die Nasenschleimhäute oder über die Atemwege vom Körper aufgenommen werden können. Über die Möglichkeit, die Medikamente genau zu dosieren hinaus ist es auch möglich, mit derartigen Spendern Medikamente zu applizieren, deren Wirkstoffdosierung kritisch sein kann, so beispielsweise Opiate. Auch Migränemittel und ähnliche Wirkstoffgruppen können mit solchen Spendern genau dosiert verabreicht werden. Solche Spender sind jedoch auch dazu geeignet, Medien mit Wirkstoffen auszutragen, die bei der Verhandlung der rheumatoiden Arthritis verwendet werden.

Derartige Spender müssen, damit sie durch einen Benutzer, beispielsweise ein Benutzer der an rheumatuider Arthritis leidet und dessen Fähigkeit zum Manipulieren von Gegenständen unter Umständen eingeschränkt ist, leicht greif- bzw. bedienbar sein. Darüber hinaus sollen die Spender einen einfachen und kostengünstigen Aufbau aus möglichst wenigen Einzelteilen besitzen.

Diese Aufgabe wird durch einen Spender mit den Merkmalen des Anspruches 1 gelöst.

Der Spender ist am Austrag von Medien, insbesondere Fluiden, beispielsweise einer Flüssigkeit geeignet, die vorzugsweise einen pharmazeutischen Wirkstoff - wie sie in beispielhafter Weise vorstehend genannt wurden - enthalten. Bei dem Spender erfolgt der Austrag des Mediums in wenigstens zwei propotionierten Teilchargen dadurch, daß ein Betätigungsmittel betätigt wird. Durch das Betätigen des Betätigungsmittels wird eine Relativbewegung zwischen dem Betätigungsmittel und einem eine Auftragsöffnung des Spenders aufweisenden Gehäuses erzeugt. Gemäß der Erfindung ist eine elastisch verformbare Materialbrücke vorgesehen. Mittels der elastisch verformbaren Materialbrücke ist das Betätigungsmittel am Gehäuse gehalten. Beim Betätigen des Betätigungsmittels im Sinne der Erzeugung eines Austrages von Medium wird die Materialbrücke durch Verformung vorgespannt.

Gemäß einer weiterführenden Ausgestaltung der Erfindung wird das Entspannen der Materialbrücke nach Abschluß der Betätigung einer betätigungsentgegengesetzer Rückhub selbsttätig erzeugt.

Es ist vorteilhaft, wenn die Materialbrücke aus zumindest einem Steg gebildet wird, der in entspanntem Zustand einen gekrümmten Verlauf hat. Dies kann, beispielsweise wenn der Steg aus einem Kunststoffmaterial besteht, durch entsprechende Formgebung bei der Herstellung erzeugt werden. Die vorgekrümmte Ausbildung ist eine einfache Erzeugung eines Elementes, daß elastisch verformbar ist. Besonders günstig sind dabei Ausbildungen, bei denen der wenigstens eine Steg in entspanntem Zustand annähernd kreisbogenförmig ist. Es können auch e-lipsoidale Formen oder dergleichen Verwendung finden. Die nahezu kreisbogenförmige Form hat jedoch den Vorteil, daß die aufzunehmenden Verformungskräfte gleichmäßig in dem Steg verteilt werden und so nicht unterschiedliche Lasten in unterschiedlichen Bereichen des Steges aufgenommen werden.

Gemäß einer bevorzugten Ausgestaltung der Erfindung sind wenigstens zwei Stege vorgesehen, wobei die Stege dann rotationssymetrisch gleichmäßig um eine Mittelachse herum verteilt sind.

Wenigstens an dem Gehäuse zugewandten Ende kann es vorgesehen sein, daß sich die Stege in einem ringförmigen Abschnitt treffen, der als Ringabschnitt bezeichnet wird. Die Stege ragen dabei insbesondere strahlenförmig von dem Ringabschnitt ab. Es ist vorteilhaft, wenn der Krümmungsverlauf der Stege so gewählt wird, daß die Stege hierbei radial in der Ebene des Ringes verlaufend ausgerichtet sind. Der Ringabschnitt ist gemäß vorteilhafter Ausgestaltung der Erfindung an dem Gehäuse des Spenders abgestützt. Er kann dazu insbesondere unlösbar mit dem Gehäuse zusammenhängend sein. Das unlösbare Zusammenhängen des Ringabschnittes mit dem Gehäuse kann beispielsweise durch Verschweißen, Verkleben oder einer anderen Form der Befestigung oder aber durch einstückige Ausbildung von Ringabschnitt und Gehäuse und der Stege erzeugt werden. Gemäß alternativer Ausgestaltungen kann es vorgesehen sein, daß der dem Gehäuse zugewandten Ringabschnitt lösbar am Gehäuse befestigt ist.

Es ist möglich, daß an dem Ringabschnitt eine Führungshülse vorgesehen ist, wobei die Führungshülse koaxial zur Mittelachse ausgebildet ist und in das Innere des Gehäuses hineinragt. Diese Hülse kann einerseits zur Befestigung des Ringabschnittes am Gehäuse dienen und andererseits auch dazu, eine Führung für innerhalb des Gehäuses anordenbare Elemente der Austragvorrichtung zu bilden. Es kann dabei insbesondere vorgesehen sein, daß diese den Verlauf einer Betätigungsbewegung des Betätigungsmittels während eines Austraghubes (Betätigungshubes) beeinflussen und/oder führen. Es kann sich dabei insbesondere um Kulissenbahnen, Führungskulissen und Rastmittel bzw. den Weg des Betätigungshubes begrenzende Mittel halten.

Gemäß einer weiterführenden Ausgestaltung der Erfindung ist die Materialbrücke als Faltenbalg ausgebildet. Dabei weist der Faltenbalg vorzugsweise einen gewendelten Faltkantenverlauf auf, so daß sich ein spiralfederkonturartiger Verlauf der Faltkante ergibt.

Gemäß bevorzugten Ausgestaltungen der Erfindung besteht die Materialbrücke aus einem Kunststoffmaterial, insbesondere aus einem buchfesten, vorzugsweise elastisch verformbaren Kunststoff. Besonders vorteilhaft ist es, wenn für die Materialbrücke das gleiche Material wie für das Gehäuse des Senders oder aber wenigstens ein Material, das in einem Mehrkomponenten-Spritzgießverfahren mit dem Material des Gehäuses verarbeitbar ist, besteht. Dabei muß bei der Konstruktion der Materialstege darauf geachtet werden, daß die Betätigungskraft beim Erzeugen eines Austraghubes des Spenders durch die vorhandenen Materialbrücken nicht unmäßig vergrößert wird. Gleichzeitig muß es möglich sein, eine hinreichende Anzahl von Lastspielen in dem Spender zu erzeugen, ohne daß eine die Funktion beeinträchtigende Änderung des Verhaltens der Materialstege eintritt. Insbesondere müssen die Materialstege auch geeignet sein, soweit elastisch verformt werden zu können, wie dies für das Erzeugen eines Austraghubes erforderlich ist. Gleichzeitig müssen die dabei entstehenden, durch Verspannen (Verformung der Materialstege) aufgebauten Kräfte ausreichend groß sein, um sicher zu stellen, daß ein Rückhub des Betätigungselementes selbsttätig erfolgt, wobei eventuell auch beim Rückhub Verastungen oder dergleichen gelöst oder überfahren bzw. überdrückt werden müssen.

Gemäß einer Ausgestaltung der Erfindung ist die Vorratskammer des Behälters, in der das Medium bevorratet ist, als Pumpenkammer einer Schubkolbenpumpe ausgebildet. Dabei ist der Schubkolben vorzugsweise als ein die Pumpenkammer dichtend verschließender Stopfen ausgebildet, wobei in dem Gehäuse ein zu der Austragsöffnung führender Austragskanal vorgesehen ist, der als Kolbenstange dient. In der ersten Betätigung wird der Stopfen dann vom entsprechend ausgebildeten Austragskanal (Nadelspitze) durchstochen und so die Verbindung zwischen Pumpenkammer und Austragsöffnung hergestellt. Anschließend wird bei der weiteren Betätigung des Spenders derart auf den Kolben eingewirkt, daß der Kolben im Sinne einer Verringerung des Volumens der Pumpenkammer derselben verfahren wird und es dadurch zum Austrag von Medium durch die Austragsöffnung des Spenders hindurch kommt.

Gemäß bevorzugter Ausgestaltung der Erfindung ist der Vorratsbehälter in einer Hülse gehalten, wobei die Hülse gegenüber dem Steuergerät verschiebbar geführt gehalten ist.

Es ist vorteilhaft, wenn der Spender so ausgebildet ist, daß der Behälter mit der Vorratskammer durch eine Öffnung in den Spender einführbar ist, wobei es sich bei der Öffnung insbesondere um einen Ringbschnitt handelt, der betätigungsmittelseitig ausgebildet ist und dessen Mittelachse vorzugsweise koaxial zur Mittelachse eines außenseitigen Ringabschnittes ausgebildet ist. Durch eine solche Öffnung, die auch abdeckbar ausgebildet sein kann, ist es möglich, zunächst den Spender wenigstens nahezu fertig herzustellen, zusammenzubauen und erst in den zusammengebauten Spender den Behälter mit dem bevorrateten Medium einzuführen. Dies ist einerseits daher von Vorteil, da dann bei dem pharmazeutischen Abfüllbetrieb keine weitere Montage des Spenders mehr vorgenommen werden muß, sondern lediglich die abgefüllten Vorratsbehälter in die Spender eingesetzt werden. Andererseits ist es dadurch auch möglich, den Spender erst unmittelbar vor der Anwendung mit einem Vorratsbehälter mit auszutragendem Medium zu versehen. Dies kann beispielsweise dann von Vorteil sein, wenn der auszutragende Wirkstoff gekühlt gelagert werden muß. In diesem Fall verringert sich das Volumen der zu kühlenden Teile auf das Behältervolumen, der Spender kann getrennt aufbewahrt werden. Es sind dabei auch Ausgestaltungen denkbar, bei denen Vorratsbehälter wieder aus dem Spender entnommen werden können und mit dem gleichen Spender und neuen, befüllten Behältern weitere definierten Teilchargen Medium ausgetragen werden können. Somit ist eine Wiederverwertbarkeit des Spenders gegeben ist. Letzteres ist insbesondere dann von Vorteil, wenn das bevorratete Medium nicht langzeitstabil ist und es daher gewünscht ist, daß das Medium beispielsweise in zwei Teilchargen ausgebracht wird, wobei für die nächsten zwei Teilchargen dann ein neuer Medienbehälter verwendet wird.

Bei einem erfindungsgemäßen Spender ist es vorteilhaft, wenn wenigstens mittelbar zwischen dem Betätigungsmittel und dem Gehäuse wirkende Rast- und/oder Anschlagmittel ausgebildet sind, die wenigstens der Begrenzung eines Betätigungshubes dienen. Zur Ausgabe von definierten Teilchargen ist es notwendig, den Betätigungsweg eines Betätigungshubes genau zu definieren. Dies kann vorteilhaft mit den entsprechenden Rast- und/oder Anschlagmitteln erreicht werden. Diese wirken nicht zwangsläufig unmittelbar zwischen dem Betätigungsmittel und dem Gehäuse. Vielmehr kann diese Wirkung auch mittelbar erzeugt werden, beispielsweise dadurch, daß der Vorratsbehälter in einer Hülse gehalten ist, das Betätigungsmittel auf den Vorratsbehälter oder die Hülse einwirkt und die Rast- und/oder Anschlagmittel zwischen dem Gehäuse und der Hülse wirkend ausgebildet sind. Über wenigstens mittelbar zwischen dem Betätigungsmittel und dem Gehäuse wirkende Rastund/ oder Anschlagmittel kann auch ein Rückhub der Pumpenkammer beim selbstätigen Rückhub des Betätigungselementes entgegengewirkt werden. Dies ist insbesondere dann notwendig, wenn es sich bei dem Spender um einen Spender mit einer Tauchkolbenpumpe handelt, dessen Pumpenkammer gleichzeitig als Vorratsbehälter wirkt.

Gemäß Ausgestaltungen der Erfindung sind wenigstens mittelbar zwischen dem Betätigungsmittel und dem Gehäuse wirkende Rastmittel vorgesehen, die zum Erzeugen eines Betätigungshubes des Betätigungselements überdrückt werden müssen. Es kann sich dabei insbesondere auch um Elemente handeln, die über Sollbruchstellen verfügen und die durch Abreißen der Sollbruchstellen den Betätigungshub freigeben. Diese Möglichkeit ist insbesondere dann vorzusehen, wenn sichergestellt werden soll, daß eine Betätigungskraft aufgebracht wird, die ausreicht, um einen Betätigungshub vollständig durchzuführen. Die Überdrückbarkeit des Rastmittels ist dann so gewählt, daß die hierfür erforderliche Kraft auch ausreicht, einen Betätigungshub des Betätigungselementes vollständig durchzuführen und der Benutzer vor dem Beenden des Betätigungshubes nicht mehr die Zeit hat, die Betätigung vor dem vollständigen Ausführen eines Austraghubes zu beenden.

Gemäß vorteilhafter Ausgestaltung der Erfindung dient die Materialbrücke selbst als Betätigungsmittel. Dies hat den Vorteil, daß eine nur sehr geringe Anzahl an Bauteilen zur Herstellung des Spenders benötigt wird.

Hierbei ist es vorteilhaft, wenn der Bereich der Materialbrücke, der an dem Gehäuse anliegt, als Gegenhalter ausgebildet ist.

Als alternative Ausgestaltungen ist es auch möglich, daß die Materialbrücke in dem nicht vom Gehäuse abgedeckten Bereich von einem Betätigungselement umschlossen ist. Diese Gestaltung hat den Vorteil, daß die Funktion der Materialbrücke nicht durch äußerliche Einwirkung auf die Materialbrücke selbst gestört werden kann. Es ist ein abgeschlossenes Gehäuse geschaffen, in dem die Materialbrücke umschlossen ist.

Damit eine der Betätigungskraft entgegenwirkende Gegenkraft einfacher Weise an dem Spender aufgebracht werden kann und der Spender zur Einhandbedienung geeignet ist, ist es vorteilhaft, wenn an dem Gehäuse einen Gegenhalter ausgebildet ist.

Die vorstehenden und weiteren Merkmale gehen außer aus den Ansprüchen auch aus der Beschreibung und den Zeichnungen hervor, wobei die einzelnen Merkmale jeweils für sich allein oder zu mehreren in Form von Unterkombinationen bei einer Ausführungsform der Erfindung und auf anderen Gebieten verwirklicht sein und vorteilhafte sowie für sich schutzfähige Ausführungen darstellen können, für die hier Schutz beansprucht wird. Diese und weiterführende Ausgestaltungen der Erfindung gehen außer aus den Ansprüchen auch aus der Beschreibung und den Zeichnungen hervor. Im übrigen ist die Erfindung auch anhand der nachfolgenden Ausführungsbeispiele dargestellt; dabei zeigt:
- Fig. 1: eine erste Ausführungsform eines erfindungsgemäßen Spenders in teilgeschnittener Darstellung;
- Fig. 2: eine teilgeschnittene Darstellung einer zweiten Ausführungsform der Erfindung;
- Fig. 3a und 3b: in unterschiedlichen Stellungen zu unterschiedlichen Betätigungszeitpunkten jeweils eine teilgeschnittene Darstellung einer dritten Ausführungsform der Erfindung;
- Fig. 4: eine vierte Ausführungsform der Erfindung in teilgeschnittener Darstellung, wobei zwischen Hülse und Gehäuse wirkende Rastmittel vorgesehen sind;
- Fig. 5: eine weitere fünfte Ausführungsform der Erfindung, eine Abwandlung der Ausführungsform gemäß Fig. 4;
- Fig. 6: eine sechste Ausführungsform der Erfindung mit einem Faltenbalg als Federelement;
- Fig. 7: eine siebte Ausführungsform der Erfindung, wobei die Materialstege durch ein Betätigungselement verdeckt werden; und
- Fig. 8: eine Abwandlung der Ausführungsform gemäß Fig. 7 mit unterschiedlicher Halterung und Führung des Behälters im Spender.

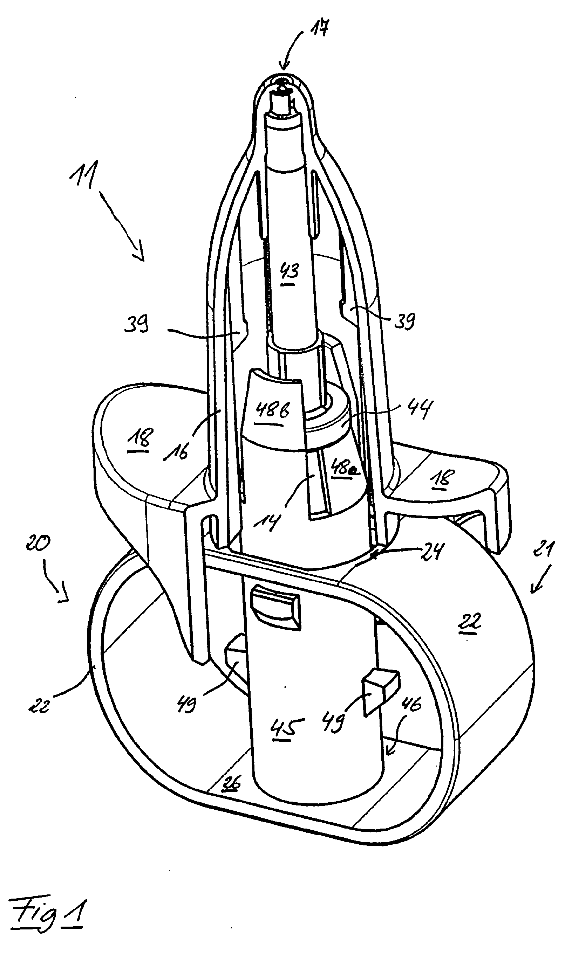

Die Fig. 1 zeigt in teilgeschnittener Darstellung eine erste Ausführungsform eines erfindungsgemäßen Spenders 11. Der Spender weist ein Gehäuse 16 auf, an dem seitlich abragend die Gegenhalter 18 ausgebildet sind. Die Austragsöffnung 17, die im Gehäuse 16 ausgebildet ist, ist mit der Vorratskammer eines Behälters 14 dadurch verbindbar, daß der Austragskanal, der in der Kolbenstange 43, die gehäuseseitig befestigt ist, ausgebildet ist mit seiner Spitze einen in dem Behälter gehaltenen Stopfen durchsticht.

In einem Ringabschnitt 24 ist das Betätigungsmittel 20, das aus einander diametral gegenüberliegenden Stegen 22, die halbkreisbogenförmig gekrümmt sind, gebildet wird, an dem Gehäuse befestigt. Dabei ist der freie Innendurchmesser des Ringabschnittes 24 um ein Aufmaß größer als der Außendurchmesser einer Hülse 45, in der der Behälter 14 gehalten ist. Dabei ist der Behälter 14 über den Behälterrand 44 hintergreifende, nach innen, dem Behälter zugewandt abgetröpfte Haltestege 48 in der Hülse gehalten. Dabei sind in der Hülse 45 erste Haltestege 48a ausgebildet, wie den Behälter 14 vor einer ersten Betätigung in der Hülse 45 gehalten, während die zweiten Haltestege 48b den Behälter vor der zweiten Betätigung halten. Dabei ist der Spender dazu bestimmt, daß in dem Behälter 14 bevorratete Medium in zwei insbesondere gleichgroß portionierte Teilchargen auszutragen.

Darüber hinaus sind an der Außenseite der Hülse Rastnasen 47 ausgebildet, die im Zusammenwirken mit dem inneren Rand des Ringabschnittes 24 dafür sorgen, daß eine Betätigung des Spenders nur dann erfolgen kann, wenn die Betätigungskraft so groß wird, daß ein der Rastwirkung der Rastnasen 47 am inneren Rand des Ringabschnittes 24 entgegenwirkendes Überdrücken stattfindet. Dabei sind die Rastnasen 47 an ihrer in der unbetätigten Ruhelage dem Ringabschnitt abgewandten Rückseite mit Schrägungen versehen, so daß bei Rückhub ein möglichst widerstandarmes Durchfahren des Ringabschnittes 24 ermöglicht wird.

Zwischen dem Ringabschnitt 24 und dem ebenfalls einem Ringabschnitt aufweisenden Boden erstrecken sich die als Stege 22 ausgebildeten Materialabschnitte des Betätigungsmittels. Dabei ist der Boden 26 als schmaler, den Ringabschnitt 46 umgebender, flächig ausgebildeter Bereich ausgebildet. Die Stege 22 sind als bandförmige Streifen ausgebildet und führen von dem Betätigungselement seitigen Ringabschitts 46 zu dem am Gehäuse anliegenden Ringabschnitt 24. Der Ringabschnitt 24 kann dabei sowohl lösbar als auch unlösbar an dem Gehäuse 16 befestigt sein. Alternativ hierzu ist es auch möglich, daß der Ringabschnitt 24 an dem Gehäuse 14 angeformt und mit diesem einschlägig verbunden ist.

Im Bereich des Bodens 26 ist, mit ihrem Innendurchmesser, dem freien Durchmesser des Ringabschnittes 46 entsprechend, die Hülse 45 an dem Betätigungsmittel 20 befestigt. Zum Betätigen des Spenders greift der Bediener den Spender, in dem er zunächst einmal Finger auf die Gegenhalter 18, die gehäuseseitig ausgebildet sind, auflegt. Der Benutzer kann dann entweder beispielsweise den Daumen auf den Boden 26 oder auch die Innenseite der Handfläche auflegen und somit den Spender 11 in einer Hand halten und dadurch betätigen, daß eine Kraft zwischen den auf den Gegenhaltern 18 aufliegenden Fingern und den am Boden 26 angreifenden Teilen der Hand aufgebracht wird. Beim Betätigen des Spenders werden zunächst einmal die bandförmigen Stege 22 dadurch verformt und vorgespannt, daß der Krümmungsradius verringert wird und damit eine stärkere Krümmung der Stege gegeben ist. Bei der ersten Betätigung des Spenders gelangen zunächst die Rastnasen 47 in Anlage mit dem Ringabschnitt 24. Die Haltestege 48a hintergreifen den Behälterrand 44 des Behälters 14. Dadurch wird der Behälter 14 in der weiteren Bewegung der Hülse 45 von dieser mitgeführt. Sobald die Betätigungskraft an dem Betätigungsmittel 20 so groß wird, daß die Rückhaltewirkung der Rastnasen 47 am Behälterrand 24 überdrückt wird, wird die Hülse 45 durch die Betätigung weiter in Richtung auf die Austragsöffnung 17 in dem Gehäuse 17 bewegt. Durch den Haltesteg 48a hintergriffen wird der Behälter 14 gleichzeitig weiter in Richtung auf die Austragsöffnung 17 bewegt. Der als Kolbenstange 43 ausgebildete, innerhalb des Gehäuses angeordnete Rohrkörper taucht in das Innere des Behälters 14 ein. Die an seinem unteren Ende ausgebildete Nadel durchsticht den in Behälter 14 gehaltenen und diesen verschließenden Stopfen. In der weiteren Bewegung wird die Kolbenstange 43, die in ihrem Inneren einen Austragskanal aufweist, weiter in den Behälter eintauchen und den dort verschiebbar gehaltenen Stopfen nach unten verschieben. Das Ende der Bewegung ist dann erreicht, wenn die Anschläge 49 in Anlage mit dem Ringabschnitt 24 gelangen und so ein Weiterbewegen der Hülse 45 in Richtung auf die Austragöffnung 17 verhindern. Während dieser Bewegung wird der Stopfen in dem als Pumpenkammer ausgebildeten Gehäuse 14 weiter nach unten verschoben, so daß sich das Volumen der Pumpenkammer verringert. Durch die Volumenverringerung verdrängte Fluid wird gelangt durch den Austragkanal hindurch zur Austragöffnung 17 und wird dort beispielsweise zerstäubt ausgetragen. Während des Ablaufes dieser Bewegung wird die Materialbrücke 21, die aus den Stegen 20 und den Ringabschnitten 24 und 26 gebildet ist, durch Verformen vorgespannt.

Sobald die Betätigung beendet ist und die auf das Betätigungsmittel 20 einwirkende Betätigungskraft zurückgeht, erfolgt ein selbsttätiger Rückhub der Hülse 45, dadurch, daß die Materialstege es anstreben, wieder in ihre Ausgangslage zurückzugehen und dabei die gespeicherte Verformungsenergie umgesetzt wird. Dabei gleitet die Hülse gegenüber dem Behälter 14 nach hinten, da der Behälter 14 über die Reibungskräfte, die zwischen der Behälterinnenwand und den Führungselementen der Kolbenstange 43 wirken, sowie über die Kräfte die zwischen Behälterinnenwand und Stopfen sowie der Nadel des Austragskanals und dem Stopfen wirken, in seiner Teilbetätigungs-Endlage gehalten wird. Die nach innen ragenden vorderen Haltestege 48b gleiten über den Behälterrand 44 nach hinten zurück und hintergreifen diese für die nächste Betätigung. Die Rastnasen 47 werden wieder in eine Stellung, die in Austragsrichtung gesehen, hinter den Ringabschnitt 24 liegt, verfahren. Der Behälter ist nun bereit, für einen zweiten Austrag von Medium, bei dem insbesondere eine gleichgroße Portion Medium ausgetragen werden kann, wie bei dem ersten Austrag. Der Austragvorgang an sich erfolgt in gleicher Weise wie der erste Austrag, nur daß ein Durchstechen des Stopfens selbstverständlich nicht mehr erforderlich ist, da dies bereits geschehen ist. Darüber hinaus wird nunmehr der Behälter 14 durch die Haltestege 48b hintergriffen und nach innen geschoben. Dabei gelangt der Behälterrand 44 in eine Lage, in der er die Greifelemente 39 nach vorne überfährt. Aufgabe der Greifelemente 39 ist es, nach der erfolgten zweiten und letzten Betätigung des Spenders 11 den Behälter 14 so zu halten, daß dieser nicht mehr nach hinten zurückgefahren werden kann und somit der Eindruck entstehen könnte, es sei ein weiterer Austrag mittels des Spenders noch möglich.

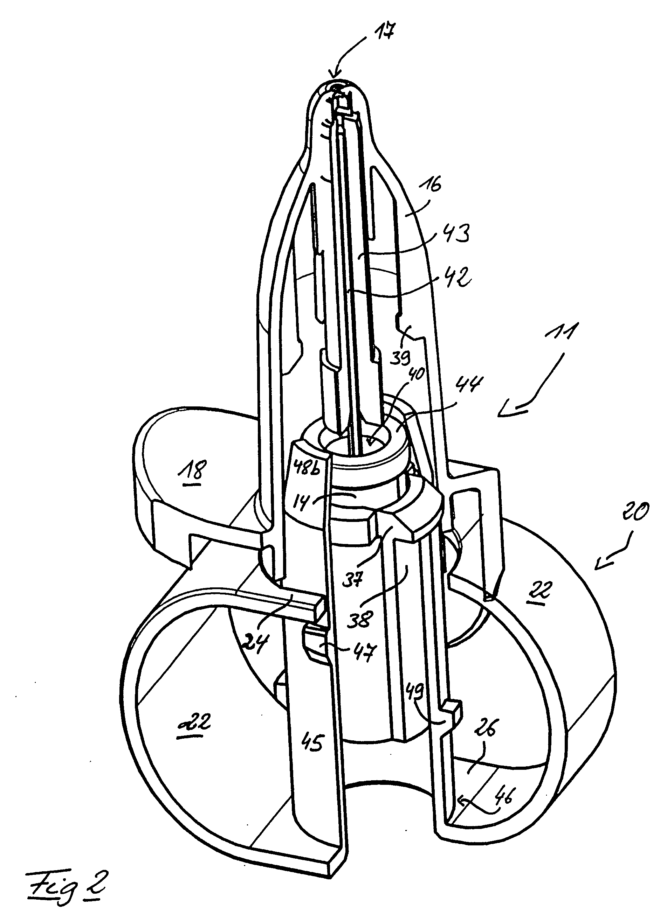

Die Fig. 2 zeigt ebenfalls in teilgeschnittener Darstellung eine zweite Ausführungsform eines erfindungsgemäßen Spenders, dabei entspricht die zweite Ausführungsform im wesentlichen der ersten Ausführungsform, so daß nachfolgend im wesentlichen nur noch auf die Unterschiedsmerkmale in Bezug auf die Ausführungsform gemäß der Fig. 1 eingegangen wird und darüber hinaus auf die Beschreibung zu Fig. 1 verwiesen wird. Im übrigen sind in der Fig. 2 auch die Hülse 45 und die Kolbenstange 43 mit dem Austragkanal 42 in teilgeschnittener Darstellung gezeigt, die in dieser Form so auch in der Ausbildung gemäß Fig. 1 ausgebildet sind.

In der Ausführungsform der Fig. 2 weist der Spender 11 ebenfalls ein Gehäuse 16 auf, das eine Austragöffnung 17 besitzt, zu der der Austragkanal 42, der innerhalb der Kolbenstange 43, einer Schubkolbenpumpe ausgebildet ist, führt. Im Inneren des Gehäuses sind ebenfalls Greifelemente 39 ausgebildet, die den Behälter 14 mit dem am Behälterrand 44 hintergreifen, nachdem der letzte portionierte Teilaustrag erfolgt ist. Auch dieser Ausführungsform sind an der Hülse 45 die Anschläge 49 ausgebildet, die als Anschlag und Hubbegrenzung für den Hubweg eines Betätigungshubes dienen und die mittelbar zwischen dem Betätigungselement, nämlich dem Betätigungsmittel 20, die als Stege 22 ausgebildet sind und dem Gehäuse wirken. Darüber hinaus sind als Rastmittel die Rastnasen 47 vorgesehen, die wenigstens mittelbar zwischen dem Betätigungselement 20 und dem Gehäuse 16 wirken und deren Wirkung überwunden werden muß, um einen Betätigungshub des Spenders zu erzeugen.

Im Unterschied zur Ausführungsform gemäß der Fig. 1 ist nicht nur eine Hülse 45 vorgesehen, in die die Vorratskammer 15, also der Behälter 14 einführbar ist, sondern zwischen der Hülse 45 und dem Behälter 14 ist darüber hinaus auch noch ein Ringkörper 38 angeordnet. Der Ringkörper 38 dient einerseits der Aufnahme des Behälters 14. Andererseits kann der Ringkörper 38 mit dem eingeführten Behälter 14 auch leicht in den Spender 11 eingeführt werden, der ansonsten vormontiert bzw. fertiggestellt ist. Um den Ringkörper in der Hülse 45 zu halten, weist dieser Rastmittel 37 auf. Die Rastmittel 37 rasten mit einer entsprechenden Ausnehmung bzw. mit der Oberkante der Hülse 45 und verhindern somit zuverlässig ein Herausnehmen des Behälters 14, sei es, daß das Herausnehmen beabsichtigt oder unbeabsichtigt erfolgt. Andererseits ist dies auch eine einfache Möglichkeit der Montage der Fixierung für den Behälter.

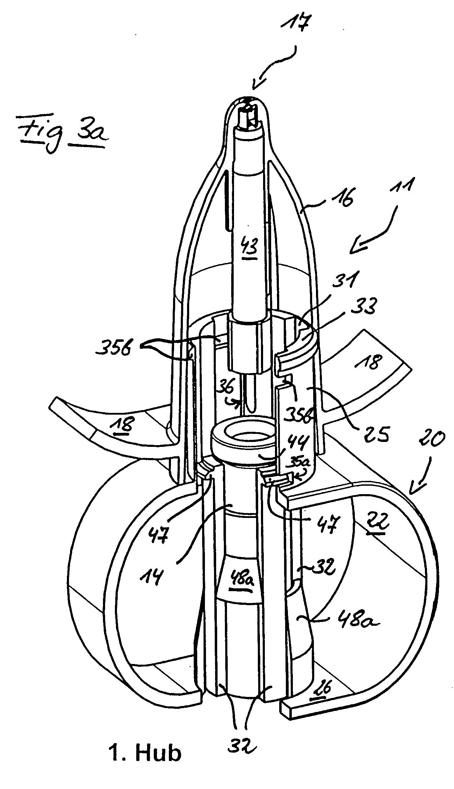

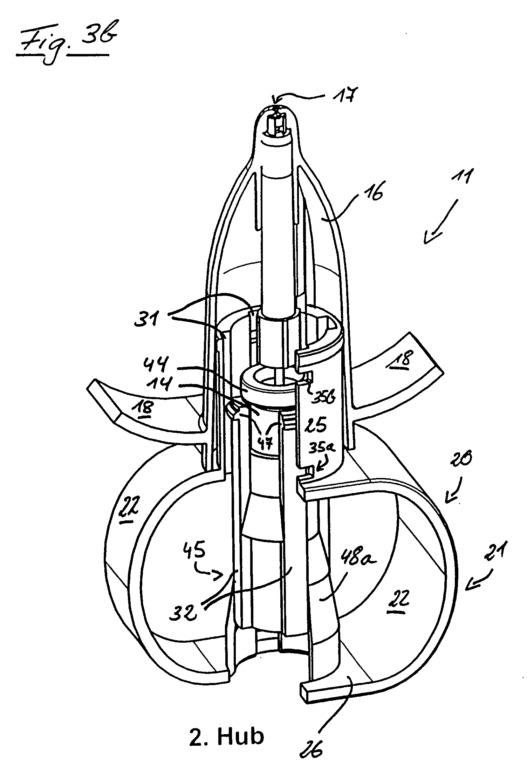

In den Fig. 3a und 3b ist ebenfalls in teilgeschnittener Darstellung eine dritte Ausführungsform der Erfindung dargestellt, die wiederum in den wesentlichen Merkmalen den Ausführungsformen gemäß den Fig. 1 und 2 entspricht. Deshalb wird auch diesbezüglich zunächst auf die Beschreibung der Fig. 1 verwiesen, wobei auch hier nachfolgend im wesentlichen noch die Unterschiedsmerkmale aufgeführt sind.

Auch bei dieser Ausführungsform weist der Spender 11 ein Gehäuse 16 auf. Innerhalb des Gehäuses führt eine Kolbenstange mit Austragskanal, der die Austragöffnung wird angeordnet. Die Kolbenstange 43 ist wiederum die Kolbenstange einer Schubkolbenpumpe, wobei der Austragskanal an seiner hinteren, der Austragöffnung 17 abgewandten Seite als Nadelspitze 36 ausgebildet ist und damit einen Stopfen, der in dem Behälter zum Verschließen desselben angeordnet ist, durchstechen kann. Am Gehäuse sind wiederum seitlich abragende Gegenhalter 18 ausgebildet, die sich durch ihre nach oben geschwungene Form sehr gut dazu eignen, als Auflage für jeweils einen Finger zu wirken, so daß der Spender am Besten zwischen Zeige- und Mittelfinger gehalten werden kann, wobei in dieser Stellung eine Betätigung des Spenders mittels des Daumens am Boden 26 des Betätigungsmittels 20 besonders günstig ist.

Das Betätigungsmittel 20 besteht im wesentlichen aus den beiden Stegen 22, die die Materialbrücke 21 bilden. An dem gehäuseseitigen Ende sind die beiden, einander diametral gegenüberliegenden Stege 22 über einen Ringabschnitt 24 miteinander verbunden, während sie an ihrem gegenüberliegenden, der Gehäuseseite abgewandten Ende in einem Betätigungselement des seitigen Ringabschnittes 46, der im Boden 26 ausgebildet ist, münden. Koaxial zur Mittelachse des Spenders, in deren Verlauf auch der Austragkanal 42 ausgerichtet ist, ist eine von dem Ringabschnitt 24 abragende Führungshülse 25 ausgebildet, die der Befestigung, insbesondere der lösbaren Befestigung des Ringabschnittes 24 am Gehäuse 16 dient. Der Ringabschnitt weist dabei an seinem vorderen, der Austragöffnung 17 zugewandtem Ende einen Ringwulst 33 auf, der in eine entsprechende Nut im Gehäuse 16 eingreift und dadurch die Führungshülse 25 festlegt. Im Unterschied zu der in Fig. 2 dargestellten Ausführungsform und entsprechenden Ausführungsform gemäß der Fig. 1 ist der Behälter 14, der die Vorratskammer 15 aufweist, in der das austragende Medium bevorratet ist und der gleichzeitig als Pumpenkammer dient, in einer Hülse 45 gehalten, die vom Betätigungselement seitigen Ringabschnitt 46 konzentrisch zur Mittelachse nach oben abragt. Dabei stützt sich der Behälterrand 44, der als wulstförmige Erweiterung des Außendurchmessers des Behälters 14 ausgebildet sein kann, auf der Hülse 45 ab. Die Hüle 45 ist dabei vorzugsweise unlösbar zusammenhängend mit dem Betätigungsmittel 20 ausgebildet und vorzugsweise an diesem angeformt. Die Hülse besteht aus in die Führungshülse 25 hineinragenden Mantelsegmente 32 und in Bereichen zwischen den Mantelsegmenten 32 ausgebildeten, nach innen abgekröpften Haltestegen 48a, die die entsprechend ausgebildeten Schuppungen und hintergreifbaren Kanten 34 auf der Behälteraußenseite hintergreifen. Um vor der ersten Betätigung eine sichere Fixierung des Behälters 14 innerhalb des Spenders 11 zu erreichen, sind an der Außenseite der Mantelsegmente 32 in Rastmulden 35 eingreifende Rastnasen 47 ausgebildet. Die Rastmulden 35 sind dabei in der Führungshülse 25 ausgebildet. In der in Fig. 3a dargestellten Ausgangsstellung vor der ersten Betätigung greifen die Rastnasen 47 in die unteren Rastmulden 35a ein, während sie nach der zweiten Betätigung in die obere Rastmulde 35b eingreifen und somit verhindern, daß der Behälter nach dem Entleeren durch die zwei proportionierten Teilhübe wieder zurückgezogen wird, In der Zwischenstellung vor dem zweiten Hub, wie er gemäß der Fig. 3b dargestellt ist, hintergreifen die Rastnasen 47 keinerlei Rastmulde, so daß vor der zweiten Betätigung eine Überdrückung der Haltekraft, wie sie für die erste Betätigung notwendig ist, um ein Lösen der Verrastung zwischen den Rastnasen 47 und den ersten Rastmulden 35a zu erreichen, nicht erforderlich ist.

Während einer Betätigung des Spenders wird eine axiale Führung der Hülse 45 dadurch erreicht, daß die Mantelsegmente 32 in entsprechend ausgebildeten Führungsnuten 31 auf der Innenseite in der Führungshülse 26 geführt werden und so ein axiales Verdrehen der Hülse 45 gegenüber der Führungshülse 25 nicht möglich ist.

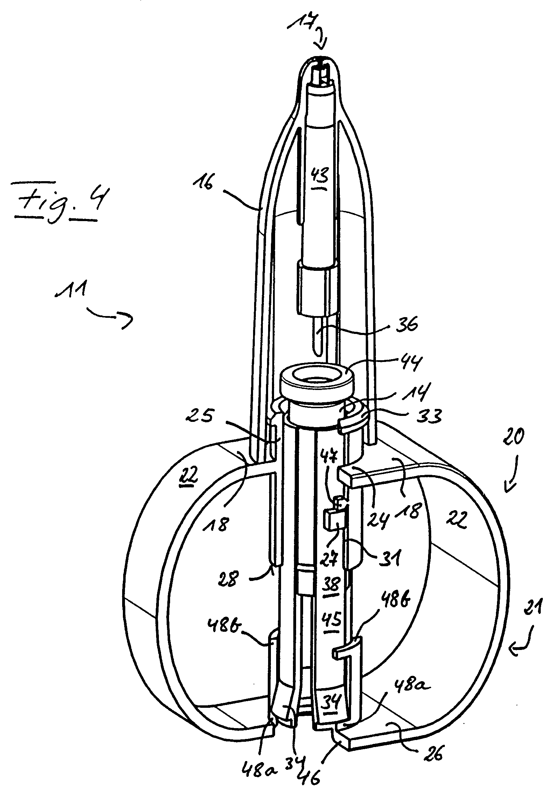

Die Fig. 4 zeigt eine vierte Ausführungsform eines erfindungsgemäßen Spenders, der wiederum eine Abwandlung der Ausführungsformen gemäß der Fig. 1 bis 3 darstellt, worauf diesbezüglich ergänzend Bezug genommen wird. In der Fig. 4 ist wiederum eine teilgeschnittene Darstellung des Spenders 11 gezeigt.

Auch bei diesem Spender ist die Austragsvorrichtung als Schubkolbenpumpe ausgebildet, wobei der Behälter selbst als Pumpenkammer dient. Von der Austragsöffnung 17 ragt im Inneren des Gehäuses 16 die Kolbenstange 43 nach innen und unten entlang der Mittelachse des Spenders ab. Im Inneren der Kolbenstange 43 ist ein Austragkanal 42 ausgebildet, dessen unteres, aus der Kolbenstange 43 herausragendes Ende als Nadelspitze 36 ausgebildet ist und zum Durchstechen eines im Inneren des Behälters 14 angeordneten, diesen verschließenden Stopfen 41 dient. Der Stopfen 41 fungiert dabei als Kolben der Austragpumpe und ist durch die über die Kolbenstange 43 auf ihn übertragenen Schubkräfte im Inneren des Behälters 14 im Sinne einer Verringerung des Volumens der Vorratskammer 15 des Behälters 14 verschiebbar und erzeugt dadurch einen Austrag von austragbarem Medium. Das Betätigungsmittel 20 ist wiederum im wesentlichen aus zwei halbkreisförmigen Stegen 22 gebildet, die als flächige Bänder ausgebildet sind und sich an ihrem oberen und unteren Ende jeweils über einen Ringabschnitt 46, 24 miteinander verbunden sind. Im Bereich des gehäuseseitigen Ringabschnittes 24 ist eine Führungshülse 25 ausgebildet, die einerseits oberhalb des Ringabschnittes 24 in das Gehäuse 16 hineinragt und dort beispielsweise mittels eines Ringwulstes 33 lösbar verrastet ist. Die Verrastung kann aber auch so ausgebildet sein, daß nach einmaligem Einschnappen der Verrastung die Verbindung nicht mehr lösbar ist. Hierzu weist dann die Ringwulst 33 eine entsprechende Formgebung auf, die in eine entsprechende Nut im Bereich des Gehäuses 16 eingreifen kann. Die Führungshülse 25 ragt jedoch auch in einem Bereich unterhalb des Ringabschnittes 25 nach unten in Richtung auf den unteren Ringabschnitt 46, der im Boden 26 ausgebildet ist, ab. Das untere Ende der Führungshülse 25 bildet dabei die Anschlagkante 28, die den Hubweg eines Betätigungshubes des Spenders 11 begrenzt. Gegen diese Anschlagkante 28 fährt beim Betätigen des Spenders die Oberkante der oberen Haltestege 48b. Die oberen Haltestege 48b und die unteren Haltestege 48a sind ähnlich einem Doppel-T-Träger ausgebildet, wobei die Doppel-T-Träger entsprechend dem Verlauf der Außenkontur der Führungshülse 25 gekrümmt sind und wobei die unteren T-Abschnitte des Doppel-T-Trägers mit den unteren Haltestegen 48a am unteren Ringabschnitt 46 des Betätigungselements 19 angeformt oder befestigt sind, während die oberen T-Abschnitte, die die oberen Haltestege 48b bilden, nach oben hin zur Anschlagkante 28 der Führungshülse 25 abragen. Der Behälter 14, der das Medium in seiner Vorratskammer 15 bevorratet, ist in einem Ringkörper 38 angeordnet. Der Ringkörper 38 ist hülsenförmig ausgebildet und weist an seinem unteren Ende nach außen hin abragende, von den Haltestegen 48a, 48b hintergreifbare Schuppungen 34 auf. Vor der ersten Betätigung des Spenders 11 wird dabei die Schuppung 34 an der unteren Kante des Ringkörpers 38 von den ersten Haltestegen 48a hintergriffen, während vor der zweiten Betätigung diese Schuppungen 34 von den Haltestegen 48 hintergriffen werden, wobei während des Rückhubes dafür gesorgt ist, daß der Ringkörper 38 nicht zurückleiten kann, während der Boden 26, von dem die Haltestege 48 abragen, aufgrund der Vorspannung der Stege 22 zurückbewegt werden und daher während des Rückhubes die vorderen Haltestege 48b zunächst hinter die Schuppungen 34 geführt werden und diese dann bei einem zweiten Betätigungshub hintergreifen.

Von dem Ringkörper 38 ragen nach außen Gleitsteine 27 ab, die in entsprechend ausgebildete Führungsnuten 31 der Führungshülse 25 geführt sind. Dabei ragen in den Verfahrweg der Gleitsteine 27 Rastnasen 47 hinein, die über Sollbruchstellen mit der Führungshülse 25 verbunden sind. Eine Betätigung des Spenders ist nur dann möglich, wenn eine Mindestbetätigungskraft aufgebracht wird, die dazu führt, daß die Rastnasen 47 an den Sollbruchstellen abgeschert werden und der Gleitstein in der Führungsnut 31 weiterverfahren werden kann. Dadurch wird sichergestellt, daß eine Mindesbetätigungskraft aufgebracht wird, die ausreicht, um einen Austragshub vollständig und ununterbrochen durchzuführen, so daß es nicht dazu kommt, daß Teilausträge vorgenommen werden, in dem bei einer Betätigung des Betätigungsmittels 20 nur ein Teilweg des möglichen Betätigungsweges zurückgelegt wird. Der Behälter 14 wird wiederum über einen Ringwulst 44 in dem Ringkörper 38 gehalten.

Dieser Spender kann in einfacher Weise dadurch hergestellt und komplettiert werden, daß zunächst der Behälter 14 in den Ringkörper 38 eingesetzt wird und dieser Ringkörper 38 dann durch den unteren Ringabschnitt 46 so weit in den Spender eingeführt wird, bis die unteren ersten Haltestege 48a die Schuppung 34 am hinteren Ende des Ringkörpers 38 hintergreifen. Dabei muß darauf geachtet werden, daß die Gleitsteine 27 sauber in die Führungsnuten 31 der Führungshülse 25 eingeführt werden. Danach kann das Gehäuse 16 auf das nunmehr mit dem Behälter 14 versehene Betätigungsmittel 20 aufgesetzt werden. Dabei hintergreift die Ringwulst 33 der Führungshülse 25 eine im Inneren des Gehäuses 16 ausgebildete Nut. Zum Betätigen des Spenders sind im Bereich des Ringabschnittes 24 ausgebildete ebene Flächen der Stege 22 als Gegenhalter 18 vorgesehen, auf dem die Finger des Benutzers aufgelegt werden können. Es handelt sich dabei vorzugsweise um Zeige- und Mittelfinger, die beiderseits des Gehäuses 16 angreifen. Das Einleiten der Betätigungskraft erfolgt durch Einleiten einer Kraft auf dem Boden 26 am anderen Ende der beiden Stege 22. Die beiden Stege 22 bilden dabei eine Materialbrücke 21 zwischen dem Boden des Betätigungselements und dem Gehäuse 16.

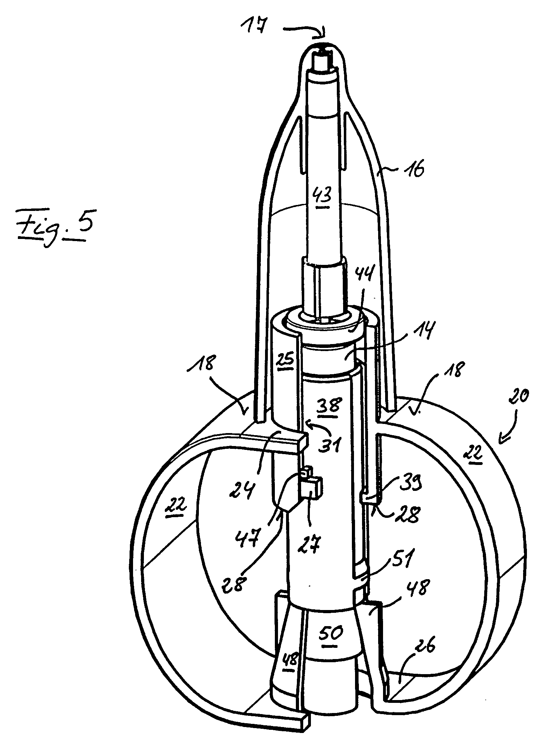

Die Fig. 5 zeigt eine fünfte Ausführungsform eines erfindungsgemäßen Spenders, wiederum in teilgeschnittener Darstellung. Die Ausführungsform gemäß der Fig. 5 ist eine Abwandlung der Ausführungsform gemäß der Fig. 4, die sich von der Fig. 4 im wesentlichen in der Art unterscheidet, wie die Kraft von dem Betätigungsmittel 20 auf den Ringkörper 38 übertragen wird. Hierzu weist nämlich der Ringkörper 38 an seinem hinteren Ende einen mindestens einmal, vorzugsweise mehrfach kegelförmigen Bereich auf, wobei die Kegelabschnitte immer kegelstumpfförmig und koaxial hintereinander angeordnet sind, wobei jeder der Kegelstümpfe 50 während einer Betätigung des Spenders 11 von dem Haltesteg 48 hintergriffen wird. Bei dem Rückhub gleitet der Haltesteg 48, der insbesondere als sich verjüngender, ebenfalls kegelstumpfförmiger, jedoch elastisch nach außen federnder Abschnitt ausgebildet sein kann, entlang der Außenkontur des nachfolgenden Kegelstumpfes 50 und hintergreift dessen untere, weiter hinten liegende Grundfläche am Ende des Rückhubes und kann somit auf den nachfolgenden Kegelstumpfabschnitt 50 die Betätigungskraft für den nachfolgenden Betätigungshub auf den Ringkörper 38 einleiten. Somit ist es möglich, nicht nur wie auch in der Fig. 5 gezeigt, zwei proportionierte Teilbetätigungen des Spenders zu ermöglichen, es kann auch eine Vielzahl weiterer Betätigungen durchführbar sein. Die Begrenzung des Betätigungsweges erfolgt wiederum durch den Abstand von der Oberkante des Haltestegs 38 zu der Anschlagskante 28 am hinteren unteren Ende der Führungshülse 25. In der Führungshülse 25 sind wiederum Führungsnuten 31 ausgebildet, in der Gleitsteine 27, die außen an dem Ringkörper 38 ausgebildet sind, verfahren werden können, wobei es wiederum möglich ist, daß an Sollbruchstellen abscherbare Rastnasen 47 in diese Führungsnuten 31 hineinragen und somit zunächst einmal die Scherkräfte zum abscheren der Rastnasen 47 aufgebracht werden müssen, was sicherstellt, daß ein durchgehender Betätigungshub durchgeführt wird, bei dem auch die notwendigen Verformkräfte aufgebracht werden, damit die Stege derart stärker gekrümmt werden, daß die Oberkante der Haltestege 48 in Anlage mit der Anschlagkante 28 gelangen und ein selbsttätiger Rückhub sicher stattfindet.

Bei der in Fig. 5 dargestellten Ausführungsform ist das Betätigungsmittel 20 nicht über die Führungshülse 25 mit dem Gehäuse 16 verbunden, vielmehr ist das Gehäuse 16 mit den Betätigungsmitteln 20 im Bereich des unteren Ende des Gehäuses 16 neben dem Ringabschnitt 24, der die Stege miteinander verbindet zusammenhängend ausgebildet. Dabei kann es sich sowohl um eine unlösbare Verbindung der beiden Teile miteinander, beispielsweise durch Verschweißen oder Verkleben, handeln als auch um eine einstückige Verbindung der beiden Bereiche miteinander.

Damit ein unerwünschtes Zurückbewegen des Ringkörpers 38 nach der letzten Betätigung des Spenders 11, die zu einem Austrag führen kann, sicher beendet wird, weist der Ringkörper 38 an seinem hinteren Abschnitt, jedoch vor den Kegelstumpfabschnitten 50 einen Sicherungssteg 51 auf, der nach der letzten Betätigung von den Greifelementen 39, die von der Fühungshülse 25 nach innen abragen und an deren hinteren Ende ausgebildet sind, hintergriffen werden und so ein Zurückbewegen des Ringkörpers 38 verhindern.

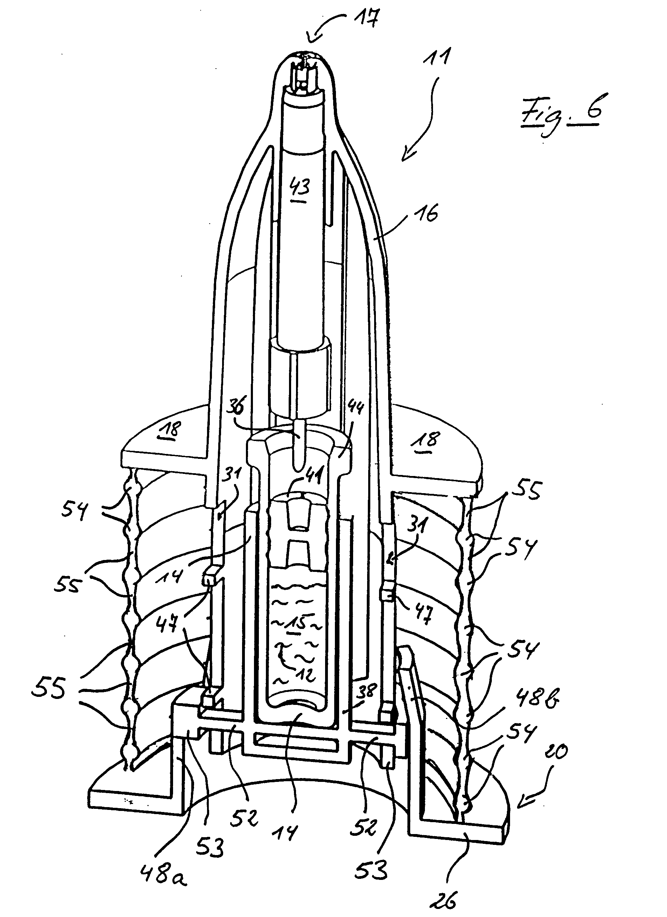

Die Fig. 6 zeigt wiederum in teilgeschnittener Darstellung, ein weiteres Ausführungsbeispiel der Erfindung. Dieses Ausführungsbeispiel unterscheidet sich dahingehend grundlegend von den Ausführungsbeispielen gemäß den Fig. 1 bis 5 sowie den Fig. 7 und 8 dadurch, daß die Materialbrücke 21 nicht in Form von Materialstegen 22, sondern in Form eines Faltenbalges 29 ausgebildet ist.

Der Spender 11 weist wiederum, entsprechend den Ausgestaltungen gemäß den Fig. 1 bis 5 ein Gehäuse 16 auf. Das Gehäuse 16 verfügt über eine Austrittsöffnung 17. Zu der Austrittsöffnung 17 führt ein Austragskanal, der mit der Vorratskammer 15 eines Behälters 14 verbindbar ist. In der Vorratskammer 15 ist beispielsweise eine Flüssigkeit 12 bevorratet, die mittels des Spenders über die Austragöffnung 17 austragbar ist. Der Behälter wird dabei durch einen Stopfen 41 verschlossen. Der Stopfen 41 verschließt dabei die Vorratskammer 15 des Behälters und ist von einer Nadelspitze 36, die austragkanalseitig ausgebildet ist, durchstechbar. Durch dieses Durchstechen des Stopfens 22 ist eine fluidische Verbindung für das auszutragende Medium zwischen der Vorratskammer 15 und der Austragsöffnung 17 herstellbar. Dabei ragt das Gehäuse 16 nach unten ab, so daß auch über die Länge des Behälters 14 hinweg das Gehäuse diesen in radialer Richtung umschließt. Dabei sind in diesem Bereich des Gehäuses Führungsnuten 31 ausgebildet, wobei in den Führungsnuten 31 an Sollbruchstellen abreißbare bzw. überfahrbare Rastnasen 47 ausgebildet sind. Im Inneren des Gehäuses wird der Behälter 14 in einem Ringkörper 38 gehalten, wobei der Ringkörper 38 axial gegenüber dem Gehäuse in Richtung auf die Austragöffnung 17 hin verschiebbar ist. Die Führungsnuten 31 durchsetzen Haltestege 52, die seitlich von dem Ringkörper 38 an dessen unteren Ende abragen. Am äußeren Ende der Haltestege 52 sind Mitnehmer 53 ausgebildet, die von den Haltestegen 48 hintergreifbar sind. Die Haltestege 48 sind von der Bodenfläche 26 im Bereich eines Ringabschnittes 46 nach oben abragend ausgebildet. Zum Betätigen des Spenders und zum Erzeugen eines Austraghubes müssen die auf den Boden 26 vom Benutzer eingeleiteten Kräfte dazu ausreichen, daß die Haltestege 52 in dem Bereich, in dem sie die Führungsnut 31 durchsetzen, die Rastnasen 47 überfahren bzw. abscheren. Im weiteren Verlauf der Führungsnut 31 angeordnete weitere Rastnasen 47 sorgen dann für einen Anschlag für die Hubbewegung, wobei die dann eingebrachte Betätigungskraft, die vermindert um die schon erzeugte Vorspannung des Faltenbalges 29 nicht mehr ausreicht, diese Sperre zu überdrücken, begrenzt. Es erfolgt dann ab dem ersten Austrag ein Rückhub, bei dem zunächst der vordere zweite Haltesteg 48b hinter den Mitnehmer 53 zurückverfahren wird und hinter diesem angreift und somit bei der nächsten Betätigung die Betätigungskraft auf diesen überträgt und somit an den Ringkörper 38 weiterleitet.

Zwischen der Unterkante des radial vom Gehäuse 16 nach außen abragenden Gegenhalters 18, der ringförmig ausgebildet ist, erstreckt sich nach unten bis zum Boden 26, der ebenfalls als Ringscheibe ausgebildet ist, der Faltenbalg 29. Der Faltenbalg 29 weist dabei einen gewendelten Faltenverlauf auf, wobei die Wendelung insbesondere in einer Materialverdickung 54 bestehen kann, wobei sich zwischen zwei Materialverdickungen 54 immer ein dünnerer Materialbereich 55 erstreckt und so ein geschlossener Faltenbalg gebildet wird, der durch ein nach innen, zum Behälter 14 hin zu erfolgendes Einknicken und Verformen der dünneren Bereiche 55 seine Länge verkürzen kann und dabei eine Vorspannung entsteht, die für einen selbsttätig erfolgenden Rückhub am Ende eines Betätigungs3hubes sorgt. Hierzu muß das Material, aus dem der Faltenbalg hergestellt ist, es kann sich dabei insbesondere um einen Kunststoff handeln, eine ausreichende Elastizität und gleichzeitig eine hinreichende Steifigkeit aufweisen. Um den Effekt der Elastizität und Steifigkeit zu verstärken, kann dabei im Inneren des Faltenbalges 29, insbesondere im Inneren der Verdickungen 54 eine metallene Spiralfeder, beispielsweise aus Federstahl, verlaufen, wobei die Verdickungen 54 selbst noch nach Art einer Spiralfeder ausgebildet sind und aus einem anderen Kunststoff hergestellt sein können wie die dünneren Wandabschnitte 55, und für den Federeffekt sorgen können. Es ist auch eine Ausgestaltung eines solchen Spenders denkbar, bei dem der Faltenbalg 29 durch ein Spiralfederelement ersetzt wird, wobei es sich dabei sowohl um eine aus Kunststoff hergestellte Spiralfeder, als auch eine aus Federstahl hergestellte Spiralfeder handeln kann. Wichtig dabei ist allerdings, daß die Spiralfeder geeignet sein muß, das Betätigungsmittel 20, daß im wesentlichen aus dem Boden 26 gebildet wird, an dem Gehäuse 19, insbesondere an der Unterkante der Gegenhalter 18, befestigt zu halten. Diese Halterung kann dabei beispielsweise durch Verkleben oder Verschweißen hergestellt werden. Hierzu wird insbesondere der Faltenbalg zunächst an der Unterkante der Gegenhalter 18 angeschweißt und anschließend nach dem Einbringen des Behälters 14 in den Ringkörper 38 und dem Einsetzen des Ringkörpers in die Führungsnuten und damit in das Gehäuse 16, der Boden 26 aufgesetzt und anschließend die noch freie Seite des Faltenbalges 29 mit dem Boden 26 des Betätigungsmittels 20 verschweißt.

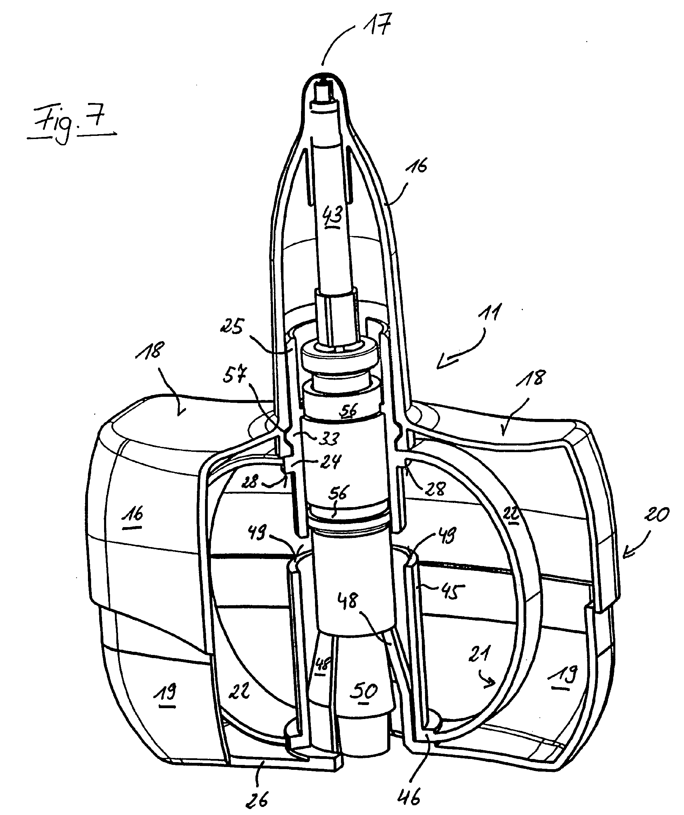

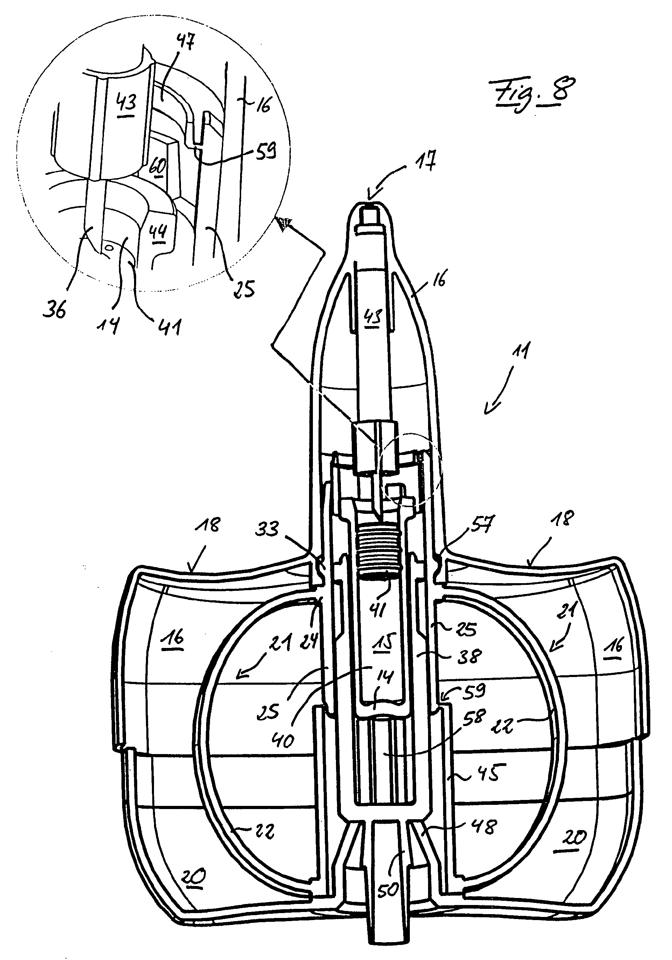

Die Fig. 7 und 8 zeigen wiederum Ausführungsformen eines erfindungsgemäßen Spenders, die im wesentlichen Abwandlungen der in den Fig. 1 bis 4 dargestellten Ausführungsformen sind, weshalb ergänzend auf die Beschreibungsteile dieser Figuren Bezug genommen wird.

Im Unterschied zu den Ausführungsformen gemäß den Fig. 1 bis 4 weisen die Spender 11 gemäß den Fig. 7 und 8 an den Betätigungsmittel 20 ein Betätigungselement 19 auf, das die Materialbrücke 21, die aus Stegen 22 gebildet ist und die das Gehäuse 19 mit dem Betätigungsmittel 20 verbinden, in den Bereichen abdeckt, in denen das Gehäuse 16 diese nicht abdeckt, so daß ein nach außen hin geschlossener nur flächige Elemente aufweisender Körper des Spenders 11 ausgebildet ist.

Das Betätigungsmittel 20 ist mit samt seinem Betätigungselement 19 mittels der Führungshülse 25 im Gehäuse 16 befestigt. Hierzu weist die Führungshülse 25 an ihrer Außenseite eine Ringwulst 33 auf, die mit einer entsprechenden Haltenut 57 im Gehäuse in Eingriff gelangen kann. Je nach Gestaltung von Ringwulst 33 und Haltenut 57 kann diese Verbindung sowohl lösbar als auch unlösbar sein. Ergänzend oder ersetzend hierzu kann anstelle der Ringwulst 33 und der Haltenut 57 auch eine Verklebung oder Verschweißung der beiden Teile miteinander vorgesehen sein. In Anlage mit dem unteren Ende des Gehäuses 16 befindet sich der gehäuseseitige Ringabschnitt 24, von dem die zwei bandförmigen Stege 22 abragen. Die bandförmigen Stege sind näherungsweise halbkreisförmig gekrümmt und durch Verformung der Stege, nämlich durch Vergrößerung des Krümmungsradiuses wird eine Vorspannung bei der Betätigung des Spenders 11 erzeugt. An dem dem Gehäuse abgewandten Ende der Stege 22 sind diese über den betätigungsmittelseitigen Ringabschnitt 46 miteinander verbunden. Von diesem Betätigungsmittel seitigem Ringwulst 46 ist das Betätigungselement 19 befestigt. Die Befestigung ist insbesondere unlösbar, beispielsweise durch Verschweißen oder Verkleben hergestellt. Von dem unteren, betätigungselementseitigem Ringabschnitt 46 ragt im Inneren der Stege 22 die Hülse 45 nach oben in Richtung auf den gehäuseseitigen Ringabschnitt 24 ab. Der gehäuseseitige Ringabschnitt 24 ist an seiner Unterseite als Anschlagkante 28 ausgebildet, wobei durch den Abstand zwischen der Anschlagkante 28 und der oberen Kante der Hülse 45, die den Gegenanschlag bildet, der Hubweg eines Betätigungshubes begrenzt ist.

Im Inneren der Führungshülse 25 ist ein Ringkörper 38 angeordnet, in dem der Behälter 14 gehalten ist. An der Unterkante des Ringabschnittes 38 sind Kegelstümpfe 50 angeformt, die von Haltestegen 48, die nach innen abragend ausgebildet sind und die Kegelstümpfe konzentrisch umgeben, hintergreifbar sind. Dabei wird zunächst die Hülse 38 soweit in den Spender eingeführt, daß die Unterseite des Ringkörpers 38 von den Haltestegen 48 hintergriffen wird. Bei der Betätigung des Spenders an dem Betätigungselement 19 wird das Betätigungselement 19 in Richtung auf die Austragsöffnung 17 bewegt. Von den Haltestegen 48 hintergriffen wird der Ringkörper 38 nach oben in Richtung auf die Austragsöffnung geschoben. Bei dieser Bewegung wird die Hülse 14 mitgeführt, so daß zunächst die Nadelspitze 36, den den Behälter 14 verschließenden Stopfen 41 durchsticht und damit die fluidische Verbindung zwischen Austragkanal 42 und der Vorratskammer 15 des Behälters 14 herstellt. Durch das im weiteren erfolgende Verschieben des Stopfens 41 in dem Behälter 14 wird das Volumen der Vorratskammer 15 verringert, es erfolgt ein Austrag des Mediums durch den Austragskanal 42 hindurch zur Austragsöffnung 17, wo insbesondere ein zerstäubender Austrag des Mediums erfolgt. Am Ende des Betätigungshubes erreicht die obere Kante 49 der Hülse 45 den Anschlag 28, eine weitere Betätigung ist nicht möglich und über diesen freien Weg wird das Volumen eines Austraghubes und somit die auszustragende Portion an Medium bestimmt. Während dieser Betätigung werden die Stege 22 verformt. Durch die elastische Verformung der Stege 22, die also einen Kraftspeicher darstellen, wird Energie aufgenommen, die dazu benutzt wird, eine selbsttätige Rückstellung des Betätigungselements 19 in seine Ausgangsstellung herzustellen. Dabei gleiten die Haltestege 48 über die Außenfläche der Kegelstümpfe soweit nach hinten, daß die Unterseite des nachfolgenden Kegelstumpfes von den Haltestegen 48 hintergriffen wird. Daher muß die Länge eines Kegelstumpfes 50 auf den Hubweg eines Betätigungshubes abgestimmt sein. Bei diesem Rückhub wird die Lage des Ringkörpers 38 bezüglich der Austragöffnung 17 nicht mehr verändert, so daß bei einer nachfolgenden Betätigung wiederum eine Verringerung des Volumens der Vorratskammer 15 entsprechend dem Hubweg des Spenders durchgeführt wird. Somit ist gewährleistet, daß bei jeder Betätigung des Spenders ein gleichgroßes Volumen, also eine gleichgroße Portion an Medium ausgetragen wird.

In der Ausgestaltung gemäß der Fig. 7 ist zum Zwecke der Fixierung des Ringkörpers 38 im Inneren der Führungshülse 25 an der Führungshülse ein als nach innen abragender Ring ausgebildete Rastnase 47 vorgesehen, die den Ringnuten 56 eingreift, die jeweils um den Weg eines Betätigungshubes voneinander beabstandet sind. Durch den Eingriff der Rastnasen 47 in die Ringnut 56 wird erreicht, daß der Ringkörper 38 beim Rückhub nicht mehr mit zurückgeführt werden kann und somit die Relativlage zwischen dem Behälter 14 und der Austragöffnung 17 im Gehäuse 16 nicht verändert wird. Darüber hinaus sorgen die Rastnasen 47 im Zusammenwirken mit der Ringnuten 56 auch dafür, daß zum Überfahren der Ringnuten 56 auf den Ringkörper 38 eine Betätigungskraft aufgebracht werden muß, die ein Mindestmaß überschreitet. Diese Betätigungskraft wird dabei so gewählt, daß bei einer derart erfolgenden Betätigung stets ein kompletter Austragshub, der durch den Hubweg begrenzt ist vorgenommen wird und nicht nur Teilbetätigungen, bei denen nicht der gesamte Hubweg des Spenders ausgenutzt wird und somit nicht eine komplette Portion des portionierten Austrages ausgetragen wird.

Bei der Ausführungsform gemäß der Fig. 8 ist gegenüber der Ausführungsform der Fig. 7 nur weniges geändert. Zum einen ist die teilgeschnittene Darstellung unter Verwendung einer anders verlaufenden Schnittlinie durchgeführt worden. Daher ist in der Fig. 8 auch ersichtlich, wie der Stopfen 41 die Vorratskammer 15 des Behälters 14 verschließt und daß der Behälter 14 auch geeignet ist, gleichzeitig die Pumpenkammer 40 der Austragsvorrichtung zu bilden. Es ist darüber hinaus in dieser Zeichnung auch ersichtlich, wie der Behälter 14 in dem Ringkörper 38 gehalten ist, wobei der Ringkörper 38 hier tassenförmig ausgebildet ist und einen abschließenden Boden aufweist. Von dem Boden können Verkürzungsstücke 58 nach oben abragen, auf denen der Behälter 14 dann aufsteht, dies geschieht dazu, daß man mit möglichst wenig konstruktiven Änderungen unterschiedliche Volumina der Behälter 14 verwenden kann, wobei in diesem Fall auch der Abstand zwischen Oberkante 49 und Anschlagkante 28 der Hüle 45, die als Anschlag dient, angepaßt werden muß.

In der Fig. 8 ist auch zu erkennen, wie die Haltestege 48 für eine erste Betätigung den Ringkörper 38 hintergreifend in einer weiteren Betätigung dann zunächst den ersten und dann gegebenenfalls weitere Kegelstümpfe 50 hintergreifen können. In der Darstellung gemäß der Fig. 8 ist zwar nur ein Kegelstumpf ausgebildet, der hintergriffen werden kann, so daß nur zwei Austraghübe mit gleichgroßen, proportionierten Medienausträgen durchgeführt werden können, über eine Vergrößerung der Anzahl der Kegelstümpfe kann jedoch auch der Austrag einer größeren Anzahl von proportionierten Austragshüben ermöglicht werden, wobei sich dann die Länge eines Kegelstumpfes ebenso entsprechend verringert, wie sich die Länge des entsprechenden Betätigungshubes an die Größenanzahl der Teilausträge anpassen muß. Das Volumen ausgetragener Menge bei einer Teilbetätigung kann dabei im wesentlichen aufgrund der Vorgaben des Betätigungshubweges aus den konstruktiven Gesichtspunkten dadurch variiert werden, daß der Innendurchmesser des Behälters 14 vergrößert wird und somit die Vorratskammer 15 des Behälters 14 ein größeres Volumen bei gleichem Hubweg eines Betätigungshubes aufweist.

Im Gegensatz zu der Ausgestaltung gemäß der Fig. 8 ist die Hülse 45 vor der Durchführung eines ersten Betätigungshubes über eine Sollbruchstelle 59 mit der Führungshülse 25 verbunden. Die Sollbruchstelle weist dann, wenn eine ausreichende Betätigungskraft auf das Betätigungselement 19, einer Greifschale aufgebracht wird.

In der herausgezogen gezeichneten vergrößerten Teildarstellung der Fig. 8 ist zu sehen, wie mittels der als ringförmiger Körper ausgebildeten Rastnase 47, die ebenfalls über eine Sollbruchstelle 59 mit dem Gehäuse 16 verbunden ist, eine Schwellkraft für die zweite Betätigung des Spenders erzeugbar ist. Der zweite Austraghub des Spenders kann erst dann erfolgen, wenn mittels des Stößels 60, der mittelbar mit dem Behälter 14 verbunden ist, durchstoßen wird. Hierzu ist das Aufbringen einer ausreichenden großen Betätigungskraft auf das Betätigungselement 19 erforderlich. Dann reißt die Sollbruchstelle 59 durch und die ringförmig ausgebildete Rastnase 47 kann im Gehäuse 16 nach oben in Richtung auf die Austragsöffnung verschoben werden.

In der vergrößerten Teildarstellung ist auch noch der Stopfen 41 ersichtlich, der in dem Behälter 14 gehalten ist, an dessen oberen Ende einen Behälterrand 44 ausgebildet ist. Der Stopfen wird von der in diesem Bereich als Nadelspitze 36 ausgebildeten Austragkanal durchstochen, sobald die erste Betätigung des Spenders erfolgt.

Ein Vorteil der Ausführungsform gemäß den Fig. 7 und 8 ist darin zu sehen, daß die Stege 22, die die Materialbrücke bilden, in einem abgeschlossenen Raum untergebracht sind, der entweder durch das Gehäuse 16 selbst oder aber durch das Betätigungselement 19 nach außen hin abgeschlossen ist. Somit hat der Benutzer keine Zugriffsmöglichkeit auf die Stege 22 und kann somit keine Beschädigung oder Blockierung dieser wichtigen Funktionselemente herbeiführen. Für die Ausbildung dieses Merkmales ist nicht relevant, ob die Materialbrücke 21 tatsächlich in Form von bandförmigen Stegen 22 ausgebildet ist oder ob es sich dabei z.B. um einen Faltenbalg, wie er beispielsweise in der Fig. 6 dargestellt ist, ausgebildet ist. Die Vorteile einer geschlossenen Ausbildung ergeben sich in letzterem Fall ebenso.

Insgesamt betrachtet sind in den Fig. 1 bis 8 unterschiedliche Ausführungsformen eines gleichen Funktionsprinzips dargestellt, wobei jeweils nur Abwandlungen in dem Bewegungsablauf und in der Gestaltung von dem Bewegungsablauf beeinflussenden Elementen in Form von Kulissenführungen, Anschlagkanten, Halteelementen, Hintergreifungen und unterschiedliche Möglichkeiten der Krafteinleitungen dargelegt wurden. Es sind selbstverständlich auch noch weitere Ausgestaltungen denkbar, die sich der Erfindung zugrunde legen und das beanspruchte Funktionsprinzip nutzbar machen.

## Patentansprüche

1. Spender für Medien, insbesondere für wenigstens einen pharmazeutischen Wirkstoff enthaltende Fluide, vorzugsweise Flüssigkeiten, wobei durch Betätigen eines Betätigungsmittels das in einer Vorratskammer bevorratete Medium in wenigstens zwei portionnierten Teilcharchen austragbar ist, wobei das Betätigen des Betätigungsmittels durch Erzeugen einer Relativbeweigung zwischen dem Betätigungsmittel und einem eine Austragsöffnung des Spenders aufweisenden Gehäuse erfolgt, **dadurch gekennzeichnet, daß**
eine elastisch verformbare Materialbrücke (21) vorgesehen ist, mittels derer das Betätigungselement (19) am Gehäuse (16) gehalten ist, wobei die Materialbrücke (21) beim Betätigen durch Verformung vorgespannt wird.

2. Spender nach Anspruch 1, **dadurch gekennzeichnet, daß** beim Entspannen der Materialbrücke (21) ein der Betätigung entgegengesetzter Rückhub selbsttätig erfolgt, wobei die Materialbrücke (21) insbesondere aus zumindest einem Steg (22) gebildet wird, der in entspanntem Zustand einen gekrümmten Verlauf hat, wobei vorzugsweise wenigstens zwei Stege (22) rotationssymmetrisch gleichmäßig verteilte um eine Mittelachse herum angeordnete, vorgesehen sind.

3. Spender nach Anspruch 2, **dadurch gekennzeichnet, daß** die Stege (22) in entspanntem Zustand annähernd kreisbogenförmig sind.

4. Spender nach einem der Ansprüche 2 oder 3, **dadurch gekennzeichnet, daß** die dem Gehäuse (16) zugewandten Enden der Stege (22) sich in einem Ringabschnitt (24) treffen, der vorzugsweise dem Gehäuse (16) zugewandt und am Gehäuse (16) abgestützt ist.

5. Spender nach Anspruch 4, **dadurch gekennzeichnet, daß** der dem Gehäuse (16) zugewandte Ringabschnitt (24) unlösbar mit dem Gehäuse (16) zusammenhängend ist.

6. Spender nach Anspruch 4, **dadurch gekennzeichnet, daß** der dem Gehäuse (16) zugewandte Ringabschnitt (24) lösbar am Gehäuse (16) befestigt ist.

7. Spender nach einem der Ansprüche 2 bis 6, **dadurch gekennzeichnet, daß** an dem Ringabschnitt (24) eine Führungshülse (25) vorgesehen ist, wobei die Führungshülse (25) koaxial zur Mittelachse ausgebildet ist und in das innere des Gehäuses (16) hineinragt.

8. Spender nach Anspruch 1, **dadurch gekennzeichnet, daß** die Materialbrücke (21) als Faltenbalg (29) ausgebildet ist, der insbesondere einen gewendelten Faltenkantenverlauf aufweist.

9. Spender nach einem der Ansprüche 1 bis 8, **dadurch gekennzeichnet, daß** die Materialbrücke (21) aus einem insbesondere bruchfestem Kunststoff besteht.

10. Spender nach einem der vorhergehenden Ansprüche, **dadurch gekennzeichnet, daß** die Vorratskammer (15) des Behälter (14) als Pumpenkammer (40) einer Schubkolbenpumpe ausgebildet ist, wobei der Schubkolben ein die Pumpenkammer (40) dichtend verschließender Stopfen (41) ist, wobei in dem Gehäuse (16) ein zu der Austragsöffnung (17) führender Austragskanal in einer Kolbenstange (43) ausgebildet ist, die bei der ersten Betätigung den Stopfen (41) durchsticht und damit die Verbindung zwischen Pumpenkammer (40) und Austragsöffnung (17) herstellt und anschließend bei Betätigung auf den Kolben derart einwirkt, daß der Kolben im Sinne einer Verringerung des Volumens der Pumpenkammer (40) in derselben verfahren wird.

11. Spender nach einem der vorhergehenden Ansprüche, **dadurch gekennzeichnet, daß** der Vorratsbehälter in einer Hülse (45) gehalten ist, wobei die Hülse (45) gegenüber dem Gehäuse (16) verschiebbar geführt gehalten ist.

12. Spender nach einem der vorhergehenden Ansprüche, **dadurch gekennzeichnet, daß** der der Behälter (14) durch eine Öffnung, insbesondere durch einen betätigungselementseitigen Ringabschnitt (24) hindurch in den Spender (11) einführbar ist.

13. Spender nach einem der vorhergehenden Ansprüche, **dadurch gekennzeichnet, daß** wenigstens mittelbar zwischen dem Betätigungselement (19) und dem Gehäuse (16) Rast- und/oder Anschlagmittel (49) ausgebildet sind, die wenigstens der Hubbegrenzung eines Betätigungshubes dienen.

14. Spender nach einem der vorhergehenden Ansprüche, **dadurch gekennzeichnet, daß** wenigstens mittelbar zwischen dem Betätigungselement (19) und dem Gehäuse (16) wirkende Rastund/oder Anschlagmittel (49) ausgebildet sind, die einem Rückhub der Pumpenkammer (40) beim Rückhub des Betätigungselement (19) entgegenwirken.

15. Spender nach einem der vorhergehenden Ansprüche, **dadurch gekennzeichnet, daß** wenigstens mittelbar zwischen dem Betätigungselement (19) und dem Gehäuse (16) wirkende Rastmittel (37) ausgebildet sind, die zum Erzeugen eines Betätigungshubes des Betätigungselement (19)s überdrückt werden müssen.

16. Spender nach einem der vorhergehenden Ansprüche, **dadurch gekennzeichnet, daß** die Materialbrücke (21) selbst als Betätigungelement dient, wobei insbesondere der Bereich der Materialbrücke (21), der an dem Gehäuse (16) anliegt, als Gegenhalter (18) ausgebildet ist.

17. Spender (11) nach einem der Ansprüche 1 bis 5, **dadurch gekennzeichnet, daß** die Materialbrücke (21) im nicht vom Gehäuse (16) abgedenkten Bereich von einem Betätigungselement (19) umschlossen ist.
